# EUROPEAN PATENT SPECIFICATION

(11) **EP 3 362 058 B1**
(45) Date of publication and mention of the grant of the patent: **01.09.2021**
(21) Application number: 16739461.8
(22) Date of filing: 15.07.2016
(51) Int. Cl.: A61K 31/138, A61K 31/495, A61P 9/10

(54) **5-HYDROXYTRYPTAMINE 1B RECEPTOR-STIMULATING AGENT FOR THE TREATMENT OF MYOCARDIAL INFARCTION**
5-HYDROXYTRYPTAMIN-1B-REZEPTOR-STIMULIERENDES MITTEL ZUR BEHANDLUNG VON MYOKARDINFARKT
AGENT DE STIMULATION DU RÉCEPTEUR 5-HYDROXYTRYPTAMINE 1B POUR LE TRAITEMENT DE L'INFARCTUS DU MYOCARDE

(30) Priority: 14.10.2015 US 201562241300 P; 15.04.2016 EP 16305443
(43) Date of publication of application: 22.08.2018
(73) Proprietor: Institut Pasteur, 75015 Paris (FR); Université de Paris, 75006 Paris (FR); Groupe Hospitalier Universitaire Paris - Psychiatrie et Neurosciences, 75014 Paris (FR)
(72) Inventor: CHRETIEN, Fabrice, Bruno, 75008 Paris (FR); GAILLARD, Raphaël, 75013 Paris (FR); ROCHETEAU, Pierre, 75014 Paris (FR)
(74) Representative: Regimbeau
(86) International application number: PCT/EP2016/066936
(87) International publication number: WO 2017/063771

(56) References cited:
- WO-A1-03/029232
- WO-A2-2007/148341
- W. H. SAUER ET AL: "Effect of Antidepressants and Their Relative Affinity for the Serotonin Transporter on the Risk of Myocardial Infarction", CIRCULATION, vol. 108, no. 1, 1 January 2003 (2003-01-01), pages 32-36, XP055301354, US ISSN: 0009-7322, DOI: 10.1161/01.CIR.0000079172.43229.CD
- JIANG WEI ET AL: "Should selective serotonin reuptake inhibitors be prescribed to all patients with ischemic heart disease?", CURRENT PSAYCHIATRY REPORTS, CURRENT SCIENCE, US, vol. 6, no. 3, 1 June 2004 (2004-06-01), pages 202-209, XP009191607, ISSN: 1523-3812
- MANDY MALICK ET AL: "Desvenlafaxine reduces apoptosis in amygdala after myocardial infarction", BRAIN RESEARCH BULLETIN., vol. 109, 1 October 2014 (2014-10-01), pages 158-163, XP055301334, GB ISSN: 0361-9230, DOI: 10.1016/j.brainresbull.2014.10.012
- P J Pauwels ET AL: "Rapid Communication The 5-H:TID Receptor Antagonist GR 127,935 is an Agonist at Cloned Human 5-HTID, Receptor Sites", , 1 January 1995 (1995-01-01), page 235231, XP055301597, Retrieved from the Internet: URL:http://ac.els-cdn.com/002839089500007S /1-s2.0-002839089500007S-main.pdf?_tid=8c4 6b0d2-78c6-11e6-86d2-00000aacb361&acdnat=1 473670663_7af01f1e99aae83691eefa20481a09b5
- DOGGRELL SHEILA A: "The role of 5-HT on the cardiovascular and renal systems and the clinical potential of 5-HT modulation", EXPERT OPINION ON INVESTIGATIONAL DRUGS, INFORMA HEALTHCARE, UK, vol. 12, no. 5, 1 May 2003 (2003-05-01), pages 805-823, XP002461894, ISSN: 1354-3784, DOI: 10.1517/13543784.12.5.805
- HUSAMETTIN GUL ET AL: "Amplification of sumatriptan-induced contractions with phenylephrine, histamine and KCl in the isolated human mesenteric artery: in-vitro evidence for sumatriptan-induced mesenteric ischaemia", NAUNYN-SCHMIEDEBERG'S ARCHIVES OF PHARMACOLOGY, vol. 366, no. 3, 1 September 2002 (2002-09-01), pages 254-261, XP055301539, DE ISSN: 0028-1298, DOI: 10.1007/s00210-002-0587-1

## Description

### INTRODUCTION

The present invention is defined in the appended claims.

The present invention relates to the field of myocardial infraction (MI). It more specifically discloses an agent stimulating, either directly or indirectly, the 5-hydroxytryptamine 1B receptor, and a composition comprising said agent, for use in the treatment of a patient having a myocardial infraction (MI). The invention further discloses therapeutic and screening methods.

Myocardial infarction (MI) is the leading cause of death worldwide. It more specifically occurs when a coronary artery is occluded, leading to insufficient oxygen supply to the myocardium and resulting in death of cardiomyocytes and non-myocyte cells. Myocardial infarction thus causes the loss of cardiac tissue and scar formation, which ultimately lead to heart failure. According to the World Health Organization, heart failure initiated by MI and coronary artery disease accounts for 29% of deaths worldwide (Celermajer et al., 2012). The adult human heart has however limited regenerative capacity, so muscle loss due to MI is typically replaced by non-contractile scar tissue, initiating progressive heart failure.

Current medical management can partially ameliorate the extent and consequences of such an infarct, and, at present, cardiac transplantation represents the only means of replacing the lost muscle. Whole-organ transplantation is however limited by the inadequate supply of donor hearts and need subsequent immunosuppression. Therefore, in recent years, drugs that diminish the consequences of MI and cell-based therapies have attracted considerable interest as an alternate means of achieving cardiac repair (Laflamme et al., 2007), and there is a need to identify new drugs capable of repairing the consequences of myocardial infarction.

The present invention addresses the above discussed need in the art.

The inventors have indeed surprisingly and unexpectedly demonstrated for the first time that the use of fluoxetine leads to a decrease in myocardial infarct size. Mechanistically, the inventors have shown that these effects were achieved via stimulation of the 5-HT1 B receptor. Importantly, it was noted that this cardioprotective effect was associated with a decrease in fibrosis after the ischemic event. These results are very promising and pave the way to the development of new drugs that can improve the outcome after MI.

### DETAILED DESCRIPTION OF THE INVENTION

Unless stated otherwise, scientific and technical terms used in connection with the present invention shall have the meanings that are commonly understood by those of ordinary skill in the art. Furthermore, unless otherwise required by context, nomenclatures used herein, and techniques of molecular biology, cell culture, and pharmacology are those well-known and commonly used in the art. Such techniques are fully explained in the literature (see Ausubel et al., Current Protocols in Molecular Biology, Eds., John Wiley & Sons, Inc. New York, 2013; Remington: The Science and Practice of Pharmacy, 22nd ed., Mack Publishing Co., Easton, Pa., 2012).

Nevertheless, with respect to the use of different terms throughout the current specification, the following definitions more particularly apply.

In the context of the present invention, the term "5-hydroxytryptamine receptor", "5-HT receptor", "5-HT R" or "serotonin receptor", refers to a superfamily of single-polypeptide seven-transmembrane receptors, found in the central and peripheral nervous systems of almost all animals, that act through the activation of G protein signaling pathways (i.e. GPCRs) and/or as ligand-gated ion channels (i.e. LGICs). 5-hydroxytryptamine receptors are activated by their natural ligand, serotonin, in order to modulate the release of many neurotransmitters, such as glutamate, GABA, dopamine, epinephrine/norepinephrine, and acetylcholine, as well as many hormones such as oxytocin, prolactin, vasopressin, cortisol, corticotropin, and substance P, among others.

The 5-hydroxytryptamine receptors are further categorized into 7 groups according to their G-protein coupling, among which the 5-HT1 group, which is of a particular interest in the context of the present invention.

The "5-HT1 receptor group" comprises the 5-HT1 A, B, D, E and F subtypes, which share in humans between 40 to 63% structural homology, and preferentially couples to Gai/o proteins. Activation of the 5-HT1 receptor subtypes typically elicits an inhibitory neurotransmission through activation of potassium channels, which decreases intracellular cAMP production.

Among the 5-HT1 receptor group, the "5-HT1 B receptor" ("5-hydroxytryptamine 1B receptor", "5-hydroxytryptamine receptor 1B", "5-HT-1B", "5-HT-1D-beta", "serotonin 1D beta receptor", or "serotonin receptor 1B") has been identified and characterized in 1992 by Jin et al. In humans, it is encoded by the *HTB1R* gene (NCBI RefSeq accession and version numbers NM_000863.1 and GI: 4504532; corresponding encoded protein: NCBI RefSeq accession and version numbers NP_000854.1 and GI:4504533), which is localized on chromosome 6 in position 6q13. The sequence of this receptor is highly conserved in humans, chimpanzee, Rhesus monkey, dog, cow, mouse, rat, chicken, zebrafish, *C.elegans,* and frog; and so far 135 organisms are known to have orthologs with the human gene *HTR1*B*.*

By "5-hydroxytryptamine 1B receptor (5-HT1 BR)-stimulating agent", or "5-HT1 BR-stimulating agent", it is meant herein an active agent capable of stimulating, either directly or indirectly, the activity of the 5-hydroxytryptamine 1B receptor, or in other words, capable of mimicking or enhancing the effects that are usually exerted by the natural ligand of said receptor (i.e. serotonin). A direct stimulation requires the binding of the agent to said receptor, while an indirect stimulation does not involve the binding of said agent to said receptor (i.e. it acts on the receptor via another mechanism of action).The capacity of a candidate agent to activate said receptor can be assessed by methods well-known in the art, for example by overexpressing 5-HT1 BR in cells and measuring intracellular cAMP production before and after contact of the recombinant cells with said candidate agent. Other methods for measuring the activity of 5-HT1 BR have been described in the art, and include, among others, a reverse phase high performance liquid chromatography coupled to an electrochemical detector (HPLC-ED) using an amperometric detector. Said agent can be selective for the 5-hydroxytryptamine 1B receptor, or may act not only on the 5-hydroxytryptamine 1B receptor but also on other receptors such as other 5-HT receptors.

According to the different aspects and embodiments of the invention described herein, a "subject" or "host" refers to a subject possessing a serotonergic system, said system comprising more particularly 5-hydroxytryptamine receptors, including notably the 5-hydroxytryptamine 1B receptor. Said subject thus preferably includes animals and humans.

Additional definitions are provided throughout the specification.

The present invention may be understood more readily by reference to the following detailed description, including preferred embodiments of the invention, and examples included herein.

The present inventors have discovered that the administration of fluoxetine, a well-known inhibitor of serotonin reuptake which increases serotonin levels and in turn stimulates 5-HT1 BR, in a mice model of ischemia reperfusion greatly reduced the size of myocardial infarct (by up to 31%), demonstrating a protecting effect of fluoxetine. They further demonstrated that this effect was mediated by the serotonin receptor 5-HT1BR, and that pharmaceutical delivery of a 5-HT1 BR inhibitor cancelled the effects of fluoxetine.

The present disclosure thus proposes to use agents stimulating 5-HT1 BR activity, either directly or indirectly, as novel drugs for treating myocardial infarction (MI) in patients suffering therefrom.

Thus, the present disclosure proposes a 5-hydroxytryptamine 1B receptor (5-HT1 BR)-stimulating agent, for use in the treatment of a patient suffering from myocardial infarction (MI).

More precisely, the disclosure proposes the use of a 5-hydroxytryptamine 1 B receptor (5-HT1 BR)-stimulating agent as described herein, for manufacturing a medicament intended to treat a patient suffering from myocardial infarction (MI).

In other words, the disclosure proposes a method for treating a patient suffering from myocardial infarction (MI), comprising the step of administering an effective amount of a 5-hydroxytryptamine 1B receptor (5-HT1 BR)-stimulating agent as described herein, to said subject.

The term "treating, "treatment" or "treat" as used herein means either ameliorating, or curing a deficiency, dysfunction, disease, or other deleterious process. In the context of the present invention, the treatment is performed on a subject that is suffering from myocardial infarction (MI), and thus aims to ameliorate said disease. It however does not encompass the prevention of MI in patients that do not suffer from said disease and/or that are at risk of suffering from it. Besides, the subject to be treated herein is preferably not suffering from, or is not treated for, a migraine, headache and/or a psychiatric disorder, such as depression, anxiety or bipolar disorder, to name a few.

By "effective amount", it is meant herein that the agent is administered in a quantity sufficient to provide the effect for which it is indicated, i.e. a reduction in size of the myocardial infarct, including the possible disappearance of said infarct. As well-known to the skilled person in the art, the term "infarct" refers to a localized area of ischemic necrosis produced by anoxia following occlusion of the arterial supply or the venous drainage of the tissue, organ, or part thereof. A "myocardial infarct" is therefore a localized area of ischemic necrosis within the muscular tissue of the heart. The size of a myocardial infarct can be assessed according to any method well-known in the art, such as by dye transfer (e.g. histology dye transfer), dye injection, CT-scan, magnetic resonance imaging or ultrasound.

As stated above, said 5-HT1 BR-stimulating agent can either act directly or indirectly on said 5-HT1 BR.

Said 5-HT1 BR-stimulating agent can be selected from the group consisting of antidepressant agents and antimigraine drugs, pharmaceutically acceptable derivatives, analogs, isomers, metabolites, salts, solvates, clathrates, polymorphs, and co-crystals thereof, and combinations thereof.

By "derivative", it is meant herein a compound that is directly derived from a chemical compound of interest (i.e. 5-HT1 BR-stimulating agent) and is structurally similar though non-identical to said compound, and which retains the same biological activity and/or physico-chemical properties.

By "analog", or "functional analog", it is meant herein a compound that is not directly derived from a chemical compound of interest and is thus structurally different, but exhibits the same biological activity and/or physico-chemical properties, such as isosters.

"Derivatives" and "analogs" of the 5-HT1 BR-stimulating agents encompass herein compounds that retain the 5-HT1 BR-stimulating activity as defined above, but that do not cross the blood-brain barrier, as further described below.

By "isomer", it is meant herein a compound having the same chemical formula as a compound of interest, but a different chemical structure. This term encompasses structural isomers and stereoisomers. Should the isomer be a stereoisomer, the individual stereoisomers (enantiomers and diastereoisomers) and mixtures thereof are also useful. Some of the disclosed compounds may exist in tautomeric forms (a type of structural isomer).

By "metabolite" as used herein, it is meant any compound that is an intermediate and/or a product of metabolism. A metabolite from a chemical compound is usually formed as part of the natural biochemical process of degrading and eliminating the compound of interest in a subject to which it is administered. Examples of metabolites of antidepressant agents are provided further below.

The term "pharmaceutically acceptable salt" or "salt as used herein refers to a salt that is physiologically tolerated (i.e. non-toxic) when used in an appropriate manner, particularly when used on mammals. Pharmaceutically acceptable salts include those derived from pharmaceutically acceptable inorganic and organic acids and bases. Examples of suitable acids include hydrochloric, hydrobromic, sulfuric, nitric, perchloric, fumaric, maleic, phosphoric, glycolic, lactic, salicylic, succinic, toluene-p-sulfuric, tartaric, acetic, citric, methanesulfonic, formic, benzoic, malonic, naphthalene-2-sulfuric, benzenesulfonic, gluconic, glutamic, bis-methylenesalicylic, ethanedisulfonic, propionic, p-amino-benzoic, malic, mandelic, cinnamic, citraconic, aspartic, stearic, palmitic, itaconic and sulfamic acids, as well as theophylline acetic acids and 8-halotheophyllines such as the 8-bromotheophylline. Other acids, such as oxalic acid, while not themselves pharmaceutically acceptable, may be employed in the preparation of salts useful as intermediates in obtaining the compounds and their pharmaceutically acceptable acid addition salts. Salts derived from appropriate bases include alkali metal (e.g., sodium), alkaline earth metal, (e.g., magnesium), ammonium and N-(C1-C4 alkyl)₄⁺ salts.

The term "solvate" should be understood as meaning any form of the active agent (i.e. 5-HT1 BR-stimulating agent), in which said compound is linked through non-covalent interactions to another molecule (normally a polar solvent), including especially hydrates and alcoholates, such as methanolate. Methods of solvation are well-known in the art.

By "clathrate", it is meant herein a chemical substance consisting of a lattice or cage that entraps or contains a second type of molecule/compound of interest, and which can be used to increase the stability and solubility in water of the molecule/compound of interest. Clathrates are typically polymeric.

The term "polymorphs" means herein different crystalline forms of a compound of interest in which molecules have different arrangements and/or different molecular conformation. It includes crystalline liquid form or crystalline solid form of a compound of interest. Hydrates and clathrates can be polymorphs.

By "co-crystal", it is meant herein a crystalline structure composed of at least two components, where the components may be atoms, ions or molecules. Solvates and clathrates may be co-crystals in certain conditions.

The pharmaceutically acceptable derivatives, analogs, isomers, metabolites, salts, solvates, clathrates, polymorphs, and co-crystals as defined above are active, i.e. they exhibit a 5-HT1 BR-stimulating activity. Said activity can be assessed as described above.

It shall further be understood that the 5-HT1 BR-stimulating agents as described herein, or their derivatives, analogs, isomers, metabolites, salts, solvates, clathrates, polymorphs, and co-crystals are preferably in pharmaceutically acceptable or substantially pure form. By pharmaceutically acceptable form, it is meant, *inter alia,* having a pharmaceutically acceptable level of purity, i.e. excluding normal pharmaceutical additives, such as diluents and carriers, and any material considered toxic at normal dosage levels. Purity levels are preferably above 98%, more preferably above 99%, and even more preferably above 99.9%. In a preferred embodiment, said purity level is 99.9%.

As stated above, 5-HT1 BR-stimulating agents can be selected among antimigraine drugs, such as triptans or ergotamine. Triptans are well-known in the art as tryptamine-based drugs used in the treatment of migraines and cluster headaches, thanks to their agonistic effects on 5-HT1 BR and 5-HT1 DR. Examples of triptans include, but are not limited to, sumatriptan, rizatriptan, zolmitriptan, eletriptan, almotriptan, frovatriptan, naratriptan, avitriptan, and donitriptan. Non limitative examples of salts of said compounds are donitriptan hydrochloride, eletriptan hydrobromide, and rizatriptan benzoate.
Ergotamine: 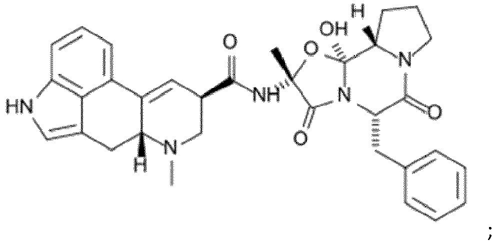
Sumatriptan: 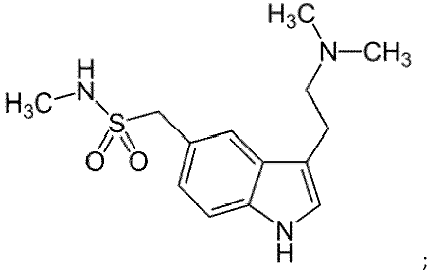
Rizatriptan: 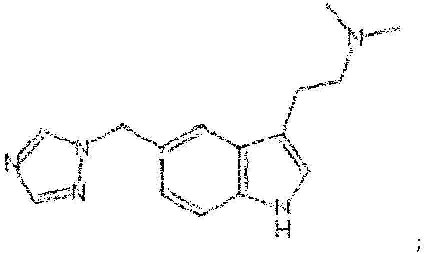
Zolmitriptan: 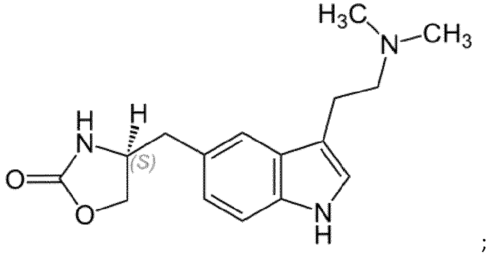
Eletriptan: 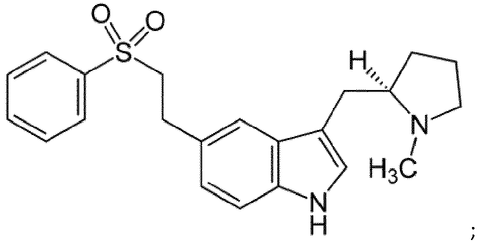
Almotriptan: 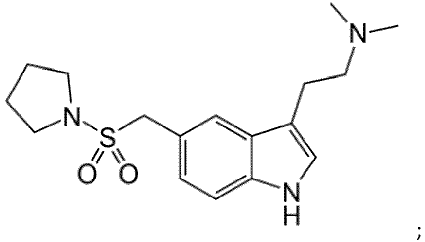
Frovatriptan: 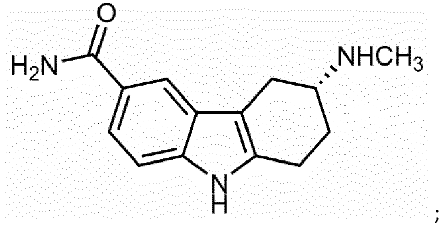
Naratriptan: 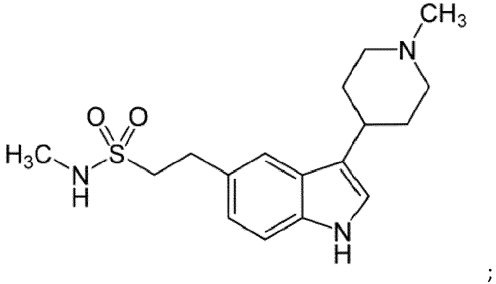
Avitriptan : 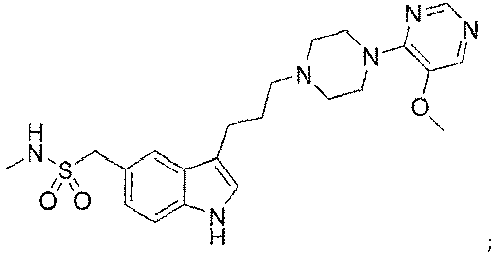
Donitriptan:

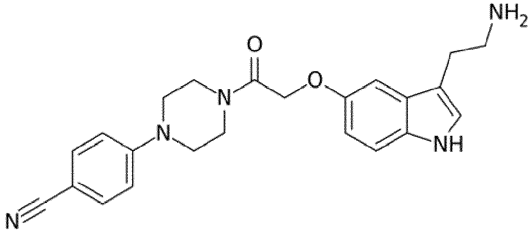

Particular triptans can be selected from the group consisting of sumatriptan, rizatriptan, zolmitriptan, eletriptan, almotriptan, and frovatriptan.

The 5-HT1 BR-stimulating agents can alternatively be selected among antidepressant agents.

By "antidepressant agent", it is meant herein an active agent that is capable to treat mood disorders, such as depression (including severe depression) and/or dysthymia. Antidepressant agents include, without limitation, serotonin reuptake inhibitors (SRIs); tricyclic antidepressants (TCAs); monoamine oxidase inhibitors (MAOs); noradrenergic and specific serotoninergic antidepressants (NaSSAs); atypical antidepressants or antidepressant enhancers.

Serotonin reuptake inhibitors (SRIs) designate a class of compounds that typically act by inhibiting the reuptake of the serotonin neurotransmitter into the presynaptic terminal, thereby increasing the serotonin extracellular level and thus serotoninergic transmission. Such compounds can act selectively or non-selectively on the neurotransmitter serotonin. SRIs can indeed also display various degrees of selectivity towards the other monoamine reuptake systems, in particular the transporters for norepinephrine and dopamine. SRIs typically include selective serotonin reuptake inhibitors (SSRIs), serotonine and norepinephrine reuptake inhibitors (SNRIs) and serotonin-norepinephrine-dopamine reuptake inhibitor (SNDRIs).

Examples of selective serotonin reuptake inhibitors (SSRIs) include, without limitation, fluoxetine, citalopram, escitalopram, sertraline, norsertraline, paroxetine, fluvoxamine, femoxetine, indalpine, alaproclate, cericlamine, ifoxetine, zimelidine, dapoxetine, and etoperidone, preferably fluoxetine, citalopram, escitalopram, sertraline, norsertraline, paroxetine, fluvoxamine, femoxetine, indalpine, alaproclate, cericlamine, ifoxetine, and zimelidine.
Fluoxetine: 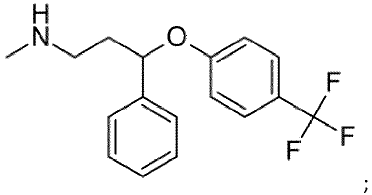
Citalopram:

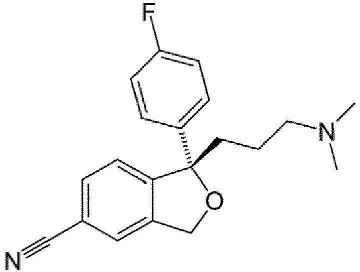

Examples of active SSRIs metabolites include, without limitation, desmethylcitalopram, didesmethylcitalopram, and seproxetine (i.e. (S)-norfluoxetine).

Examples of serotonine and norepinephrine reuptake inhibitors (SNRIs) include, without limitation, duloxetine, venlafaxine, desvenlafaxine, milnacipran, levominalcipran, and sibutramine.

Examples of serotonin-norepinephrine-dopamine reuptake inhibitor (SNDRIs) (also known as triple reuptake inhibitor or TRI) include, without limitation, bicifadine, brasofensine, tesofensine and nomifensine, preferably bicifadine.

Examples of tricyclic antidepressants (TCAs) include, without limitation, clomipramine, amoxapine, nortriptyline, maprotiline, trimipramine, imipramine, desipramine and protriptyline.

Examples of monoamine oxidase inhibitors (MAOs) include, without limitation, iproniazide, phenelzine, tranylcipromine, moclobemide, selegiline and rasagiline.

Examples of noradrenergic and specific serotoninergic antidepressants (NaSSAs), acting preferably by blocking presynaptic alpha-2 adrenergic receptors, include, among others, mirtazapine, mianserin, aptazapine, esmirtazapine, setiptiline and S32212 (also known as N-[4-methoxy-3-(4-methylpiperazin-1-yl)phenyl]-1,2-dihydro-3H-benzo[e]indole-3-carboxamide), preferably mirtazaine and mianserin.

Examples of atypical antidepressants (defined as such as they do not belong to any of the foregoing classes of antidepressants) or antidepressant enhancers include, without limitation, bisarylsulfanyl amines such as vortioxetine, as well as tianeptine, agomelatine, nefazodone, trazodone, buspirone, tandospirone, and ketamine, preferably vortioxetine, tianeptine, agomelatine, nefazodone, trazodone, buspirone, tandospirone, and ketamine.

Bisarylsulfanyl amines have been disclosed in patent application WO 2003/029232 . Said compounds can be described according to the following general formula (A): 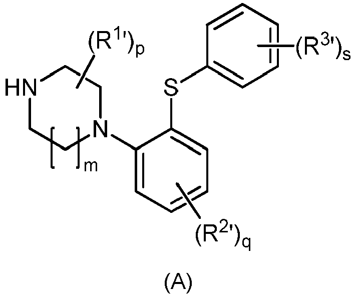 wherein
- m is 1 or 2;
- p is 0, 1, 2, 3, 4, 5, 6, 7 or 8;
- q is 0, 1, 2, 3 or 4;
- s is 1 or 2;
- each R^{1'} is independently selected from the group represented by C₁₋₆-alkyl, or two R^{1'} attached to the same carbon atom may form a 3-6-membered spiro-attached cycloalkyl;
- each R^{2'} is independently selected from the groups represented by halogen, cyano, nitro, C₁₋₆-alk(en/yn)yl, C₁₋₆-alk(en/yn)yloxy, C₁₋₆-alk(en/yn)ylsulfanyl, hydroxy, hydroxy-C₁₋₆-alk(en/yn)yl, halo-C₁₋₆-alk(en/yn)yl, halo-C₁₋₆-alk(en/yn)yloxy, C₃₋₈-cycloalk(en)yl, C₃₋₈-cycloalk(en)yl-C₁₋₆-alk(en/yn)yl, acyl, C₁₋₆-alk(en/yn)yloxycarbonyl, C₁₋₆-alk(en/yn)ylsulfonyl, or -NRxRy; -NRxCO-C₁₋₆-alk(en/yn)yl;
- each R^{3'} is independently selected from a group represented by halogen, cyano, nitro, C₁₋₆-alk(en/yn)yl, C₁₋₆-alk(en/yn)yloxy, C₁₋₆-alk(en/yn)ylsulfanyl, hydroxy, hydroxy-C₁₋₆-alk(en/yn)yl, halo-C₁₋₆-alk(en/yn)yl, halo-C₁₋₆-alk(en/yn)yloxy, C₃₋₈-cycloalk(en)yl, C₃₋₈-cycloalk(en)yl-C₁₋₆-alk(en/yn)yl, C₁₋₆-alk(en/yn)ylsulfonyl, aryl, C₁₋₆-alk(en/yn)yloxycarbonyl, acyl, -NR^{x}CO-C₁₋₆-alk(en/yn)yl, CONR^{x}R^{y} or NR^{x}R^{y};
   or two adjacent R^{3'} substituents together form a heterocycle fused to the phenyl ring selected from the group consisting of 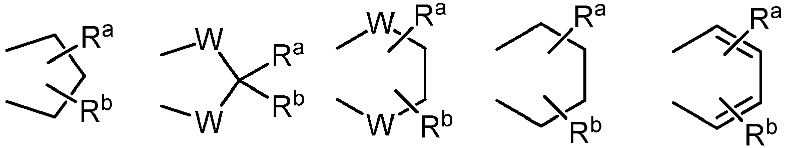
   wherein W is O or S, and R^{a} and R^{b} are hydrogen or C₁₋₆-alkyl; or two adjacent R^{3'} substituents together form a fused heteroaromatic system containing one, two or three heteroatoms,
   wherein each R^{x} and R^{y} is independently selected from the group represented by hydrogen, C₁₋₆-alk(en/yn)yl, C₃₋₈-cycloalk(en)yl, C₃₋₈-cycloalk(en)yl-C₁₋₆-alk(en/yn)yl, or aryl; or R^{x} and R^{y} together with the nitrogen to which they are attached form a 3-7-membered ring which optionally contains one further heteroatom;
   or a pharmaceutically acceptable salt thereof.

Synthesis of compounds of general formula (A) is fully described in WO 2003/029232 and therefore does not need to be detailed herein.

A preferred embodiment of general formula (A) is wherein p is 0.

A preferred embodiment of general formula (A) is wherein m is 1 or 2.

A preferred embodiment of general formula (A) is wherein R^{2'} is trifluoromethyl, or C₁₋₆-alkyl.

A preferred embodiment of general formula (A) is wherein R^{3'} is selected from the group consisting of halogen, C₁₋₆-alkoxy, C₁₋₆-sulfanyl, C₁₋₆- alkyl hydroxy and trifluoromethyl.

A more preferred embodiment of general formula (A) is wherein m=1, p=0, q=0, R^{3'} is methyl and s=2.

Particularly preferred embodiment of general formula (A) is wherein the compound of formula (A) is any of the following:
1-[2-(2-Trifluoromethylphenylsulfanyl)phenyl]piperazine, 1-[2-(4-Bromophenylsulfanyl)phenyl]piperazine, 1-{2-[4-(Methylsulfanyl)phenylsulfanyl]phenyl}piperazine, 1-[2-(4-Hydroxyphenylsulfanyl]phenyl}piperazine, 1-[2-(2,4-Dimethylphenylsulfanyl)phenyl]piperazine, also known as vortioxetine
   Vortioxetine: 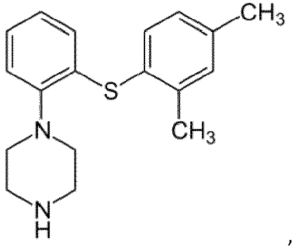
1-[2-(3,5-Dimethylphenylsulfanyl)phenyl]piperazine,
1-[2-(2,6-Dimethylphenylsulfanyl)phenyl]piperazine,
1-[2-(2,5-Dimethylphenylsulfanyl)phenyl]piperazine,
1-[2-(2-Trifluoromethylphenylsulfanyl)phenyl][1,4]diazepane,
1-[2-(3-Methylphenylsulfanyl)phenyl]-[1,4]-diazepane,
2-(4-Methylphenylsulfanyl)phenyl-1-piperazine,
1-[2-(4-Chlorophenylsulfanyl)phenyl]-piperazine,
1-[2-(4-Methoxyphenylsulfanyl)-4-chlorophenyl]piperazine,
1-[2-(4-Methoxyphenylsulfanyl)-4-methylphenyl]piperazine,
1-[2-(4-Methoxyphenylsulfanyl)-5-methylphenyl]piperazine,
1-[2-(4-Fluorophenylsulfanyl)-5-methylphenyl]piperazine,
1-[2-(4-Methoxyphenylsulfanyl)-5-trifluoromethylphenyl]piperazine,
1-[2-(4-Chlorophenylsulfanyl)phenyl]-3-methylpiperazine,
1-[2-(4-Chlorophenylsulfanyl)phenyl]-3,5-dimethylpiperazine,
   or a pharmaceutically acceptable salt thereof.

Most preferred embodiment is wherein the compound of formula (A) is 1-[2-(2,4-Dimethylphenylsulfanyl)phenyl]piperazine (i.e. vortioxetine).

"Halogen" means herein fluoro (F), chloro (CI), bromo (Br) or iodo (I).

"Alkyl", "alkenyl", "alkynyl", and "aryl" are further defined below.

The expression C₁₋₆-alk(en/yn)yl means a C₁₋₆-alkyl, C₂₋₆-alkenyl or a C₂₋₆-alkynyl group.

The expression C₃₋₈-cycloalk(en)yl means a C₃₋₈-cycloalkyl- or cycloalkenyl group.

The term C₁₋₆ alkyl refers to a branched or unbranched alkyl group having from one to six carbon atoms including but not limited to methyl, ethyl, 1-propyl, 2-propyl, 1-butyl, 2-butyl, 2-methyl-2-propyl and 2-methyl-1-propyl.

Similarly, C₂₋₆ alkenyl and C₂₋₆ alkynyl, respectively, designate such groups having from two to six carbon atoms, including one double bond and one triple bond respectively, including but not limited to ethenyl, propenyl, butenyl, ethynyl, propynyl and butynyl.

The term C₃₋₈ cycloalkyl designates a monocyclic or bicyclic carbocycle having three to eight C-atoms, including but not limited to cyclopropyl, cyclopentyl, cyclohexyl.

The term C₃₋₈ cycloalkenyl designates a monocyclic or bicyclic carbocycle having three to eight C-atoms and including one double bond.

In the term C₃₋₈-cycloalk(en)yl-C₁₋₆-alk(en/yn)yl, C₃₋₈-cycloalk(en)yl and C₁₋₆-alk(en/yn)yl are as defined above.

The terms C₁₋₆-alk(en/yn)yloxy, C₁₋₆ alk(en/yn)ylsulfanyl, hydroxy-C₁₋₆-alk(en/yn)yl, halo-C₁₋₆-alk(en/yn)yl, halo-C₁₋₆-alk(en/yn)yloxy, C₁₋₆-alk(en/yn)ylsulfonyl etc. designate such groups in which the C₁₋₆-alk(en/yn)yl are as defined above.

As used herein, the term C₁₋₆-alk(en/yn)yloxycarbonyl refers to groups of the formula C₁₋₆-alk(en/yn)yl -O-CO-, wherein C₁₋₆-alk(en/yn)yl are as defined above.

As used herein, the term acyl refers to formyl, C₁₋₆-alk(en/yn)ylcarbonyl, arylcarbonyl, aryl-C₁₋₆-alk(en/yn)ylcarbonyl, C₃₋₈-cydoalk(en)ylcarbonyl or a C₃₋₈-cydoalk(en)yl-C₁₋₆-alk(en/yn)yl-carbonyl group.

The term 3-7-membered ring optionally containing one further heteroatom as used herein refers to ring systems such as 1-morpholinyl, 1-piperidinyl, 1-azepinyl, 1-piperazinyl, 1-homopiperazinyl, 1-imidazolyl, 1-pyrrolyl or pyrazolyl, all of which may be further substituted with C₁₋₆-alkyl.

The heterocycles formed by two adjacent R^{3'} substituents and fused to the parent ring may together form rings such as 5-membered monocyclic rings such as 3*H*-1,2,3-oxathiazole, 1,3,2-oxathiazole, 1,3,2-dioxazole, 3*H*-1,2,3-dithiazote, 1,3,2-dithiazole, 1,2,3-oxadiazole, 1,2,3-thiadiazole, 1*H*-1,2,3-triazole, isoxazole, oxazole, isothiazole, thiazole, 1*H*-imidazole, 1*H-*pyrazole, 1*H-*pyrrole, furan or thiophene and 6-membered monocyclic rings such as 1,2,3-oxathiazine, 1,2,4-oxathiazine, 1,2,5-oxathiazine, 1,4,2-oxathiazine, 1,4,3-oxathiazine, 1,2,3-dioxazine, 1,2,4-dioxazine, 4*H*-1,3,2-dioxazine, 1,4,2-dioxazine, 2*H*-1,5,2-dioxazine, 1,2,3-dithiazine, 1,2,4-dithiazine, 4*H*-1,3,2-dithiazine, 1,4,2-dithiazine, 2*H*-1,5,2-dithiazine, 2*H-*1,2,3-oxadiazine, 2*H*-1,2,4-oxadiazine, 2*H*-1,2,5-oxadiazine, 2*H*-1,2,b-oxadiazine, 2*H*-1,3,4-oxadiazine, 2*H*-1,2,3-thiadiazine, 2*H*-1,2,4-thiadiazine, 2*H*-1,2,5-thiadiazine, 2*H*-1,2,6-thiadiazine, 2*H*-1,3,4-thiadiazine, 1,2,3-triazine, 1,2,4-triazine, 2*H*-1,2-oxazine, 2*H*-1,3-oxazine, 2*H*-1,4-oxazine, 2*H*-1,2-thiazine, 2*H*-1,3-thiazine, 2*H*-1,4-thiazine, pyrazine, pyridazine, pyrimidine, 4*H*-1,3-oxathiin, 1,4-oxathiin, 4*H*-1,3-dioxin, 1,4-dioxin, 4H-1,3-dithiin, 1,4-dithiin, pyridine, 2*H*-pyran or 2*H*-thiin.

Further, the compounds of general formula (A) may exist in unsolvated as well as in solvated forms with pharmaceutically acceptable solvents such as water, ethanol and the like,

Some of the compounds of general formula (A) contain chiral centers and such compounds exist in the form of isomers (i.e. enantiomers). Such isomers and any mixtures thereof including racemic mixtures are also herein disclosed.

Particular antidepressant agents can be selected from the group consisting of fluoxetine, bisarylsulfanyl amines as described above such as vortioxetine, and citalopram, escitalopram, sertraline, paroxetine, fluvoxamine, femoxetine, indalpine, alaproclate, zimelidine, duloxetine, venlafaxine, desvenlafaxine, milnacipran, levominalcipran, sibutramine, bicifadine, clomipramine, amoxapine, maprotiline, imipramine, desipramine, moclobemide, selegiline, mirtazapine, mianserin, tianeptine, agomelatine, trazodone, buspirone, tandospirone, and ketamine. Antidepressant agents can be selected from the group consisting of fluoxetine and bisarylsulfanyl amines as described above such as vortioxetine.

Other suitable 5-HT1 BR-stimulating agents can be: anpirtoline hydrochloride, CGS-12066A, CGS 12066B dimaleate, oxymetazoline, 5-carboxamidotryptamine, CP-93129 and salts thereof such as CP-93129 dihydrochloride, CP-94253 and salts thereof such as CP-94253 hydrochloride, CP-122,288, CP-135,807, RU-24969 and salts thereof such as RU-24969 hemisuccinate, ziprasidone, asenapine, 5-nonyloxytryptamine oxalate, pindolol and (S)-(-)-pindolol.

According to a preferred embodiment, a 5-HT1 BR-stimulating agent can be an antidepressant selected from the group consisting of SRIs, preferably SSRIs, and atypical antidepressants.

The 5-HT1 BR-stimulating agent can be the SSRI fluoxetine or the atypical antidepressant vortioxetine.

Nevertheless, in the event that the 5-hydroxytryptamine 1B receptor (5-HT1 BR)-stimulating agent exerts undesired CNS-related adverse effects, it is particularly advantageous to limit the effects of said agent onto the peripheral serotonin system. Antidepressant agents are notably well-known for exerting such side effects. Side effects can be prevented by chemically modifying the structure of said agent, and by notably grafting a charged chemical moiety to prevent crossing of the blood-brain barrier.

Thus, in a particular aspect, the present invention relates to fluoxetine or a derivative thereof, for use for reducing the size of a myocardial infarct in a patient suffering from myocardial infarction, wherein said derivative is fluoxetine which is modified to comprise one or more charged chemical moieties.

Said 5-HT1 BR-stimulating agent, and in particular Fluoxetine, can be modified to comprise at least one positively charged chemical moiety. Notably, the positive charge can be retained at a wide range of pH, in particular at a physiological pH.

Such modified 5-HT1 BR-stimulating agents are not capable of crossing the blood-brain barrier. Antidepressant agents and anti-migraine drugs modified in this manner are thus, respectively, anti-depressant disabled and anti-migraine disabled.

Such chemical modifications have been extensively described in patent application WO 2007/148341, incorporated herein by reference, and can be performed so as to retain the 5-HT1 BR-stimulating activity of the compounds, while preventing them from crossing the blood-brain barrier.

The term "charged chemical moiety", "charged moiety", "charged chemical group" or "charged group", as used herein, refers to an atom or a group of atoms which forms a part of an organic molecule, and which is characterized by a positive or negative electrostatic charge.

By "positively charged chemical moiety", "positively charged moiety", "positively charged chemical group" or "positively charged group", it is thus meant herein a charged chemical moiety as defined above, which is characterized by a positive electrostatic charge. Compounds which include one or more positively charged moieties are molecular ions often referred to as molecular cations. A positively charged group of atoms has at least one electron less than the number of protons in these atoms. Positively charged chemical moieties include, without limitation, ammonium and sulfonium groups.

A positively charged group which retains its charge at physiological pH is a group that is not capable of participating in proton-exchange interactions at a pH range which is typical to the physiological environment in the body where the 5-HT1-BR stimulating agent is active. Typically, the physiological pH is about 7.4; therefore a positively charged group which retains its charge at physiological pH refers to a positively charged chemical group that stays ionized in a pH range of about 5-8. It is noted that even in the GI, where the pH level is extremely low in terms of physiological pH, the positively charged chemical moiety according to the invention remains positively charged, and hence modified 5-HT1-BR stimulating agents, are not adversely affected by the GI pH levels. Still, yet, according to a further preferred embodiment, said positively charged chemical moiety is a quaternary ammonium group or a tertiary sulfonium group.

By "quaternary ammonium", it is meant herein a nitrogen atom which forms a part of a molecule (an amine) that is attached to four non-hydrogen substituents and thus is positively charged. The term "amine" describes a -NR'R" group where each of R' and R" is independently hydrogen, alkyl, cycloalkyl, heteroalicyclic, aryl or heteroaryl.

By "tertiary sulfonium group", it is meant herein a sulfur atom which forms a part of a molecule (a sulfonium) that is attached to three non-hydrogen substituents and thus is positively charged. The term "sulfonium" refers to a -S⁺R'R", wherein R' and R" are each independently alkyl, cycloalkyl, heteroalicyclic, aryl or heteroaryl.

According to the invention, the term "alkyl group" refers to a linear or branched saturated aliphatic group. Preferably, the alkyl group has 1 to 20 carbon atoms, more preferably 1-10 carbon atoms, and even more preferably between 1-6 carbon atoms. Whenever a numerical range; e.g., "1-10", is stated herein, it implies that the group, in this case the alkyl group, may contain 1 carbon atom, 2 carbon atoms, 3 carbon atoms, etc., up to and including 10 carbon atoms. Examples of alkyl groups include, without limitation, methyl, ethyl, propyl, butyl, tert-butyl and isopropyl groups. The alkyl group can be further substituted. When substituted, the substituent can be, for example, an alkyl, an alkenyl, an alkynyl, a cycloalkyl, an aryl, a heteroaryl, a halide, a hydroxy, an alkoxy and a hydroxyalkyl. The term "alkyl", as used herein, further encompasses saturated or unsaturated hydrocarbon, hence this term further encompasses alkenyl and alkynyl.

The term "cycloalkyl" refers to an aliphatic monocyclic or bicyclic ring having 3 to 8 carbon atoms, and includes, without limitation cyclopropyl, cyclopentyl, cyclohexyl, etc.

The term "alkenyl" describes an unsaturated alkyl, as defined herein, having at least two carbon atoms and at least one carbon-carbon double bond, and which can be substituted by one or more substituents, as described above. The term "alkynyl" is an unsaturated alkyl having at least two carbon atoms and at least one carbon-carbon triple bond, and which can be substituted by one or more substituents, as described above.

The term "aryl" describes an all-carbon monocyclic or fused-ring polycyclic (i.e., rings which share adjacent pairs of carbon atoms) groups having a completely conjugated pi-electron system.

By "aryl or heteroaryl group", it is meant herein a mono- or polycyclic aromatic group comprising preferably between 4 and 15 carbon atoms, preferably between 5 and 10 carbon atoms. Examples of aryl groups include, without limitation, phenyl, naphtyl, etc. The aryl group according to the invention may be further substituted by one or more substituents, as described above. Heteroaryl groups typically comprise at least one heteroatom, such as nitrogen, oxygen, and sulfur - a heteroatom being any atom that is not carbon or hydrogen. Examples of heteroaryl groups include, without limitation, pyrrole, furane, thiophene, imidazole, oxazole, thiazole, pyrazole, pyridine, pyrimidine, quinoline, isoquinoline and purine. The heteroaryl group may be further substituted by one or more substituents, as described above; representative examples are thiadiazole, pyridine, pyrrole, oxazole, indole, purine and the like.

In a more preferred embodiment, said quaternary ammonium group has the formula (I)

-(NR₁R₂R₃)⁺Z⁻ (I)

wherein
Z is an organic or inorganic anion, such as NO₃⁻, H₂PO₄²⁻, Br-, HSO₄⁻, CH₃SO₃⁻, or tartaric acid anion; and
R₁, R₂ and R₃ are each independently selected from the group consisting of alkyl, aryl and cycloalkyl.

Preferably, R₁, R₂ and R₃ are each an alkyl having from 1 to 4 carbon atoms, and more preferably, R₁, R₂ and R₃ are each methyl, resulting in the positively charged group, or the quaternary ammonium group -(NMe₃)⁺.

In another preferred embodiment, said tertiary sulfonium group has the formula (II)

-(SR₄R₅)⁺Z⁻ (II)

wherein
Z is an organic or inorganic anion, such as NO₃⁻, H₂PO₄²⁻, Br-, HSO₄⁻, CH₃SO₃⁻, or tartaric acid anion; and
R₄ and R₅ are each independently selected from the group consisting of alkyl, aryl and cycloalkyl.

Preferably, R₄ and R₅ are each an alkyl having from 1 to 4 carbon atoms, and more preferably, R₄ and R₅ are each methyl, resulting in the positively charged group, or the sulfonium -(SMe₂)⁺.

The positively charged group can be formed on the 5-HT1 BR-stimulating agent from an existing group which forms a part of the 5-HT1 BR-stimulating agent, namely, by turning a partially charged or uncharged group into a positively charged group, or by turning an existing positively charged group which can participate in proton-exchange interaction into one that cannot participate in such interaction, making it into an irreversible positive charge, or a permanent positive charge, thereby modifying the 5-HT1 BR-stimulating agent.

Alternatively, the positively charged group can be added to the 5-HT1 BR-stimulating agent by substituting one or more carbon atom with a positively charged group, e.g., by replacing a hydrogen atom or any other substituent with a quaternary ammonium or a tertiary sulfonium group.

Examples of preferred 5-HT1 BR-stimulating agents from which the compounds described herein can be derived include, without limitation, fluoxetine, bisarylsulfanyl amines as described above such as vortioxetine, as well as citalopram, alaproclate, dapoxetine, fluvoxamine, paroxetine, sertraline, venlafaxine, zimelidine, etoperidone, densvalafaxine, duloxetine, minalcipran, nefazodone, venlafaxine, brasofensine, tesofensine and nomifensine, preferably vortioxetine, fluoxetine, citalopram, alaproclate, dapoxetine, fluvoxamine, paroxetine, sertraline, venlafaxine and zimelidine. Indeed, all these agents already comprise at least one amine group, which can be readily converted into a quaternary ammonium, i.e. a positively charged group as defined above. In particular, said agents can be modified to comprise at least one quaternary ammonium group of formula (I) as described above.

An example of derivative of citalopram that comprises such a quaternary ammonium group is n-methyl-citalopram (NMC), of which the synthesis is fully detailed in patent application WO2007/128341.

Bisarylsulfanyl amines of formula (A) are also herein particularly advantageous as they comprise not only an amine group, but also a sulfur group, which can be readily converted into a quaternary ammonium group and/or into a tertiary sulfonium group, respectively. Positively charged moieties can also be attached to the carbon atom(s) of the piperazine group of said compounds.

Particularly preferred derivatives of said bisarylsulfanyl amines are compounds of formula (B) as follows: 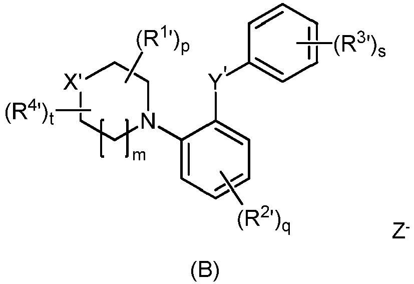 wherein
- Z is an organic or inorganic anion as defined above, such as NO₃⁻, H₂PO₄²⁻, Br-, HSO₄⁻ , CH₃SO₃⁻, or tartaric acid anion, or a mixture of organic or inorganic anions, whose global charge is such that the compound of formula (B) is neutral;
- R^{1'}, R^{2'}, R^{3'}, m, p, q and s are as defined above, wherein R^{3'} can optionally be a C₁₋₆-alk(en/yn)yloxy group substituted by an ammonium or sulfonium group as defined above, preferably R^{3'} is choline;
- t is 0, 1, 2, 3, 4, 5, 6, 7 or 8, preferably 0 or 1, more preferably 0, with the proviso that t+p ≤ 8;
- each R^{4'} is at least one charged chemical moiety, identical or different, preferably positively charged, as defined above, such as a sulfonium or an ammonium group;
- X' is selected from the group consisting of:
   ∘ -(NR^{5'}R^{6'})⁺- wherein
      R^{5'} and R^{6'} are each independently selected from the group represented by hydrogen, alkyl, aryl and cycloalkyl as defined herein, preferably by an hydrogen, a C₁₋₆-alkyl, and a C₃₋₈-cycloalkyl ; or
      R^{5'}R^{6'} form together with the nitrogen to which they are attached a cycloheteroalkyl, preferably a 3-8-membered cycloheteroalkyl, more preferably a 3-6-membered cycloheteroalkyl;
   ∘ -NH-;
   ∘ -NR^{7'}-wherein R^{7'} is a C₁₋₆-alkyl;
   ∘ -N⁺(O⁻)R^{8'}- wherein R^{8'} is a C₁₋₆-alkyl;
   ∘ -NC(O)R^{9'}- wherein R^{9'} is an amino acid, said amino-acid being preferably positively charged such as histidine, arginine or lysine, or an amino acid derivative, said derivative being preferably positively charged such as choline or carnitine, or a C₁₋₆-alkyl phosphonium;
- Y' is selected from the group consisting of:
   ∘ -S-;
   ∘ -(SR^{10'})⁺- wherein R^{10'} is selected from the group represented by hydrogen, alkyl, aryl and cycloalkyl as defined herein, preferably is a C₁₋₆-alkyl ; and
   ∘ -S⁺(O)⁻-.

The skilled person in the art would readily understand that the anions Z are present to counterbalance the positive charges on the molecule. Accordingly, compounds of formula (B) comprise as many anions Z as necessary to neutralize the positive charges of the molecule. One skilled practitioner would further understand that when p>0 and t>0, R^{1'} and R^{4'} are attached to any of the carbon atoms of the heterocyclic ring, albeit to different carbons.

In a preferred embodiment, only one of X', Y', R^{4'} (when t>0) and R^{3'} (when substituted by an ammonium or sulfonium group) is a positively charged chemical moiety.

Preferred embodiments regarding R^{1'}, R^{2'}, R^{3'}, m, p, q and s are as defined above.

The 5-HT1 BR-stimulating agent from which the compounds described herein are derived can be selected from the group consisting of SRIs, preferably SSRIs, and atypical antidepressants such as bisarylsulfanyl amines as defined above.

More preferably, the 5-HT1 BR-stimulating agent to be modified is the SSRI fluoxetine or the atypical antidepressant vortioxetine.

Most preferably, the 5-HT1 BR-stimulating agent to be modified is fluoxetine.

For example, vortioxetine can be chemically modified, as follows: 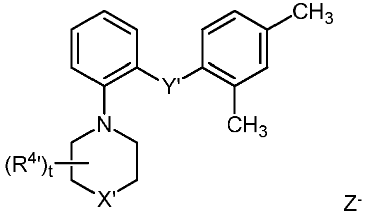 wherein Z, t, R^{4'}, X' and Y' are as defined above. More preferably, t=0.

Particularly preferred salts, derivatives and/or analogs of vortioxetine, which comprise at least one charged chemical moiety, preferably positively charged, are selected from the group consisting of:
- 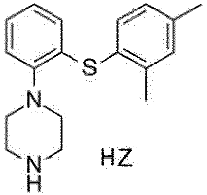 wherein HZ is preferably HNO₃, H₃PO₄, HBr, H₂SO₄, CH₃SO₃H, or tartaric acid (salts of vortioxetine);
- 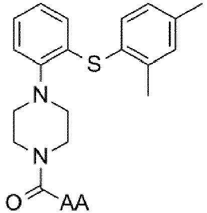 wherein AA is an amino acid, preferably 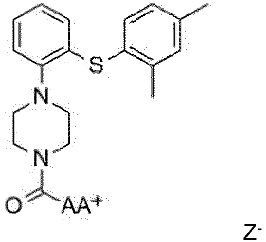 wherein AA+ is a positively charged amino acid such as histidine, arginine or lysine;
- 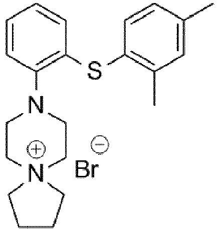 (pyrrolidinium-vortioxetine);
- 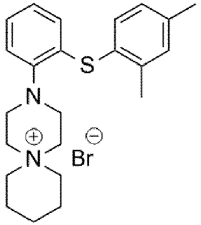 (pyperazinium-vortioxetine) ;
- 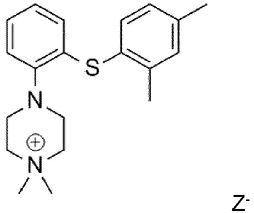 (dimethylammonium-vortioxetine);
- 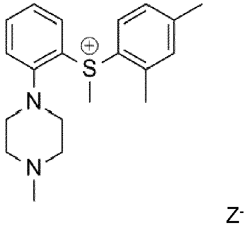 (sulfonium-vortioxetine);
- 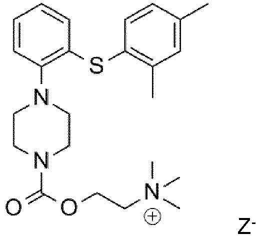 (choline-vortioxetine); 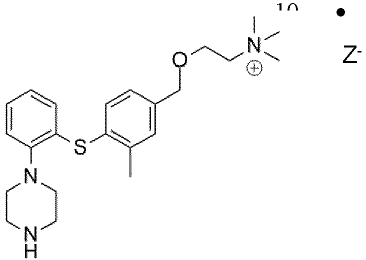 (benzyle-choline-vortioxetine);
- 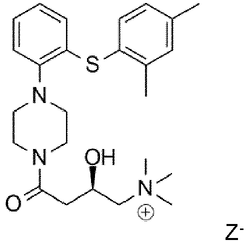 (L-carnitine-vortioxetine);
- 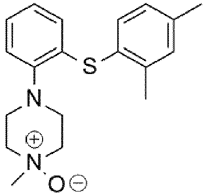 (N-oxide-vortioxetine);
- 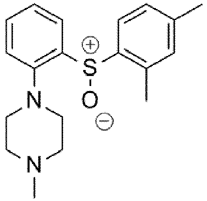 (sulfoxide-vortioxetine);
- 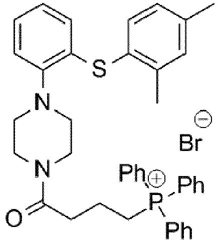 (phosphonium-vortioxetine); and
- 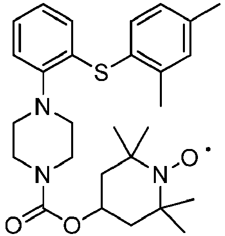 (tempol-carbamate-vortioxetine).

Preferably, said salts, derivatives and/or analogs of vortioxetine, which comprise at least one charged chemical moiety, preferably positively charged, are selected from the group consisting of:
- salts of vortioxetine as described above;
- vortioxetine coupled to a positively charged amino acid (preferably at least one) such as histidine, arginine or lysine, as described above;
- pyrrolidinium-vortioxetine;
- pyperazinium-vortioxetine;
- dimethylammonium-vortioxetine;
- sulfonium-vortioxetine;
- N-oxide-vortioxetine;
- sulfoxide-vortioxetine;
- phosphonium-vortioxetine; and
- tempol-carbamate-vortioxetine.

More preferably, said salts, derivatives and/or analogs of vortioxetine, which comprise at least one positively charged chemical moiety, are selected from the group consisting of:
- salts of vortioxetine as described above;
- vortioxetine coupled to a positively charged amino acid (preferably at least one) such as histidine, arginine or lysine, as described above ;
- pyrrolidinium-vortioxetine;
- pyperazinium-vortioxetine;
- dimethylammonium-vortioxetine;
- sulfonium-vortioxetine;
- N-oxide-vortioxetine;
- sulfoxide-vortioxetine ; and
- phosphonium-vortioxetine.

Yet, even more preferably, said salts, derivatives and/or analogs of vortioxetine, which comprise at least one positively charged chemical moiety, are selected from the group consisting of:
- salts of vortioxetine as described above;
- vortioxetine coupled to a positively charged amino acid (preferably at least one) such as histidine, arginine or lysine, as described above ;
- pyrrolidinium-vortioxetine;
- pyperazinium-vortioxetine;
- dimethylammonium-vortioxetine;
- sulfonium-vortioxetine ; and
- phosphonium-vortioxetine.

Still, even more preferably, said positively charged vortioxetine is selected from the group consisting of histidine-vortioxetine and pyrrolidinium-vortioxetine.

The above compounds can be prepared according to conventional methods in the art. Such methods are described in further details below.

For example, in order to synthetize pyrrolidinium-vortioxetine or pyperazinium-vortioxetine, one skilled person in the art can proceed as follows:

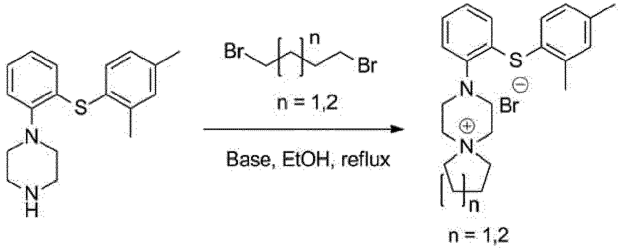

More particularly, for pyrrolidinium (n=1) formation on 4-arylpiperazine, the reaction can be performed using either K₂CO₃, ethanol (EtOH) and reflux for 10h (Mokrosz et al., 1992, incorporated herein by reference), or K₂CO₃, acetone and reflux for 15h (see WO 2004/9914A1, incorporated herein by reference).

Still, for example, in order to synthetize dimethylammonium-vortioxetine, one skilled person in the art can proceed as follows:

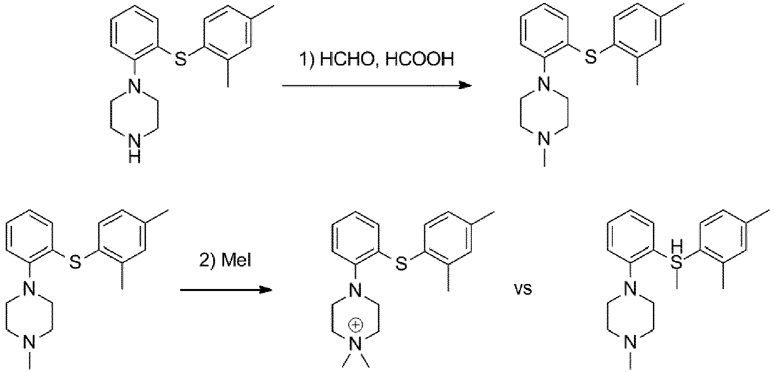

More particularly, for 4-arylpiperazine dimethylation, one can refer to Romanelli et al. (2001) (incorporated herein by reference), the first step being an Eschweiler-clarke reaction, and the second step being a methylation.

As another illustrative example, in order to synthetize amino acid derivatives of vortioxetine, choline-vortioxetine and carnitine-vortioxetine, one skilled person in the art can proceed as follows:

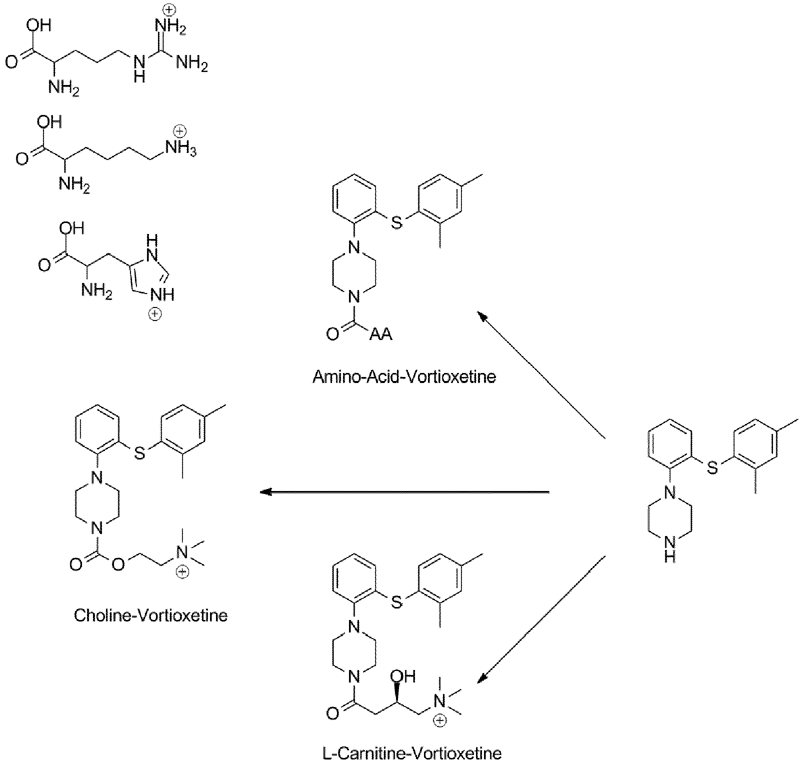

More particularly, for amide formation on the carnitine, the reaction can be performed on either acylhydrazine using pyridine, ethylene dichloride (EDC), dimethylformamide (DMF), and ethanol (EtOH) (Kuroda et al., 1996, incorporated herein by reference), or on primary amine using pyridine, ethylene dichloride (EDC), methanol (MeOH) and acetonitrile (CH₃CN) (Nakaya et al., 2001, incorporated herein by reference).

As another illustrative example, in order to synthetize phosphonium-vortioxetine, one skilled person in the art can proceed as follows:

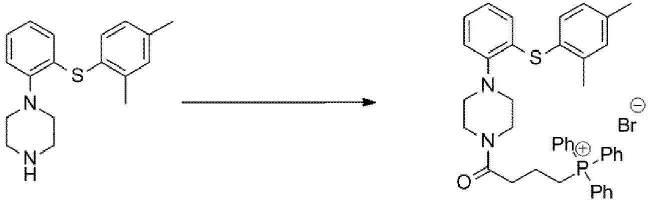

As Phosphonium-Vortioxetine another illustrative example, in order to synthetize N-oxide-vortioxetine, one skilled person in the art can proceed as follows:

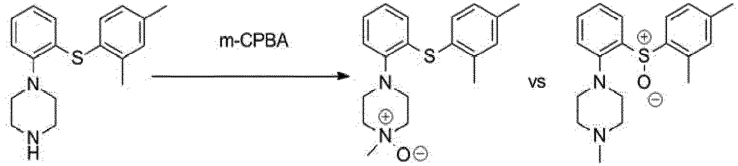

More particularly, for N-oxidation of 4-aryl-piperazines, the reaction can be performed using meta-chloroperoxybenzoic acid (m-CPBA) and CH₂Cl₂ at 20-45°C (see US 2008/153812A1, WO 2011/162515A2 or WO 2004/104007A1, incorporated herein by reference).

As another illustrative example, in order to synthetize tempol-carbamate-vortioxetine, one skilled person in the art can proceed as follows:

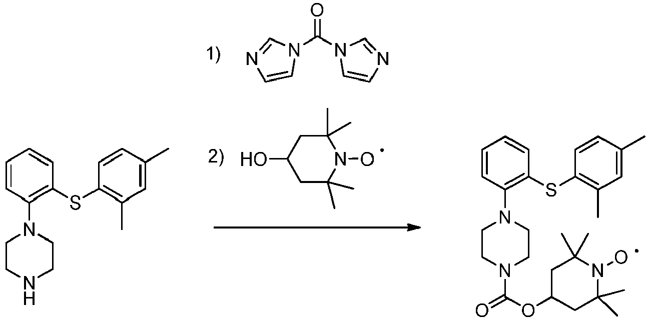

As another illustrative example, in order to synthetize benzyl-choline-vortioxetine, one skilled person in the art can proceed as follows:

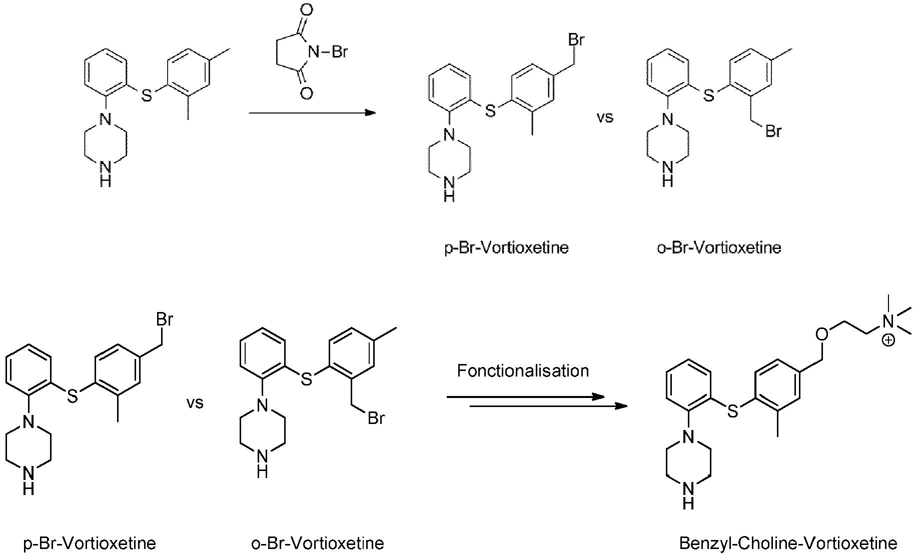

More particularly, for benzylic position bromation, the reaction can be performed using either N-bromosuccinimide, azobisisobutyronitrile (AIBN) and tetrachloromethane (CCl₄) (see US 2010/4159A1, incorporated herein by reference), or N-bromosuccinimide, meta-chloroperoxybenzoic acid (m-CPBA) and tetrachloromethane (CCl₄) (see Farmaco, 1989, 44, from p.683, incorporated herein by reference).

The 5-hydroxytryptamine 1B receptor (5-HT1 BR)-stimulating agent as defined above can be used for therapeutic purposes, in a pharmaceutical composition which may comprise additional active agent(s), or in a combined preparation which may be administered simultaneously, separately or sequentially to a subject in need thereof.

It is thus a further aspect of the invention to provide a pharmaceutical composition for use in the treatment of a subject suffering from myocardial infarction, wherein said composition comprises at least Fluoxetine or a derivative thereof as defined above, and at least one pharmaceutically acceptable excipient.

More precisely, the disclosure proposes the use of said pharmaceutical composition, for manufacturing a medicament intended to treat a subject suffering from myocardial infarction.

In other words, the disclosure proposes a therapeutic method for treating a subject suffering from myocardial infarction, comprising the step of administering an effective amount of said pharmaceutical composition to said subject.

The subject to be treated is as described above. In particular, the subject does not already suffer from MI and/or is not at risk of suffering from it (in other words, the treatment does not encompass the prevention of MI). Besides, the subject is preferably not suffering from, or is not treated for, a migraine, headache and/or a psychiatric disorder, such as depression, anxiety or bipolar disorder, to name a few.

By "pharmaceutically acceptable excipient", it is meant herein a compound of pharmaceutical grade which improves the delivery, stability or bioavailability of an active agent, and can be metabolized by, and is non-toxic to, a subject to whom it is administered. Preferred excipients according to the invention include any of the excipients commonly used in pharmaceutical products, such as, for example, microcrystalline cellulose, lactose, starch, and soybean powder.

According to a preferred embodiment, said pharmaceutical composition further comprises at least one active agent, which may either be capable of treating a myocardial infarction by itself (albeit with a possible different efficacy than the 5-HT1 BR-stimulating agent) and/or increasing or potentiating the action of 5-HT1 BR-stimulating agent(s) (i.e. increasing or potentiating the stimulation of the activity of the 5-hydroxytryptamine 1 B receptor that is exerted by the 5-HT1 BR-stimulating agent).

Examples of suitable active agents according to the invention include, without limitation, beta-blockers, antithrombotics (e.g. aspirin, anti-GP2b3a, statins, heparins and derivatives thereof, streptokinase, etc.), trinitrine, vasodilatators, angiotensin-converting-enzyme inhibitors, angiotensin-receptor blockers, L-carnitine, lidocaine (also known as 2-(diéthylamino)-N-(2,6-diméthylphényl)acétamide), calcium channel inhibitors, non-steroidal anti-inflammatory drugs (NSAIDs), therapeutic cells (e.g. enriched progenitor cells, myocardial cells such as myocardial stem cells, mesenchymal stem cells, etc.), and growth factors. Indeed, the combined preparation of the 5-HT1 BR-stimulating agent and of the above-mentioned active agents can significantly improve the reduction in size of the myocardial infarct, or even completely cure MI.

It is within the skill of ordinary person in the art to select the appropriate combination of 5-HT1 BR-stimulating agent and active agent, for the purposes of the invention.

The pharmaceutical composition of the invention may preferably be in a form suitable for the purposes of the invention. For example, said composition may be in a form suitable for parenteral, oral or topical administration, such as a liquid suspension, a solid dosage form (granules, pills, capsules or tablets), or a paste or gel. The term parenteral as used herein includes subcutaneous injection, intravenous, or intramuscular injection. For example, the pharmaceutical composition is in a form suitable for intramuscular administration, and more particularly for intracardiac administration.

As indicated above, it is particularly advantageous to combine a 5-hydroxytryptamine 1B receptor (5-HT1 BR)-stimulating agent with an active agent as described above.

It is thus another aspect of the invention to provide Fluoxetine or a derivative thereof as defined above and an active agent, as a combined preparation for simultaneous, separate or sequential administration in a subject suffering from myocardial infarction.

In other words, the disclosure proposes a method for treating a subject suffering from myocardial infarction, comprising:
a) administering to said subject at least one 5-hydroxytryptamine 1B receptor (5-HT1 BR)-stimulating agent; and
b) further administering to said subject at least one active agent, simultaneously, separately or sequentially to (i.e. before or after) step a).

The subject to be treated is as described above. In particular, the subject does not already suffer from MI and/or is not at risk of suffering from it (in other words, the treatment does not encompass the prevention of MI). Besides, the subject is preferably not suffering from, or is not treated for, a migraine, headache and/or a psychiatric disorder, such as depression, anxiety or bipolar disorder, to name a few.

The skilled person in the art would further understand that the 5-HT1 BR-stimulating agent or composition can further be administered, in association to conventional surgical therapies, including, without limitation, percutaneous coronary intervention (PCI) such as percutaneous transluminal coronary angioplasty (PTCA), coronary artery bypass grafting (CABG), mitral valve replacement with coronary bypass, coronary stenting, progenitor cell injections, and gene therapy.

The dose and scheme of administration of the pharmaceutical composition according to the invention can be adapted by the skilled person in the art depending on the age, weight and severity of the symptoms of the subject to be treated.

Accordingly, in another aspect, the composition according to the invention can be administered once a day, preferably for a period of about 6 weeks (in particular for slow-acting 5-HT1 BR-stimulating agents such as fluoxetine) or for a period of about 12 days (in particular for fast-acting 5-HT1 BR-stimulating agents such as vortioxetine).

Yet, in a preferred embodiment, the composition according the invention is administered at a dosage comprised between about 5 mg/kg and about 30 mg/kg, preferably between about 10 mg/kg and about 25 mg/kg, more preferably between about 15 mg/kg and about 20 mg/kg and preferably at 18 mg/kg.

The 5-hydroxytryptamine 1B receptor-stimulating agent may be used for drug screening purposes. In particular, novel drug assays may be provided, which identify therapeutics treating, in an efficient manner, myocardial infarction.

The disclosure more particularly proposes an *in vitro* screening method for identifying an agent or combination of agents treating myocardial infarction, comprising the steps of:
a) contacting a biological sample comprising a myocardial infarct with a candidate agent or combination of candidate agents ;
b) assessing said biological sample ;
c) comparing the sample in step b) to one in the absence and/or presence of at least one 5-hydroxytryptamine 1B receptor (5-HT1 BR)-stimulating agent as described above.

In another aspect, the present invention relates to an *in vitro* screening method for identifying an agent or combination of agents treating myocardial infarction, comprising the steps of:
a) contacting a biological sample comprising a myocardial infarct with a candidate agent or combination of candidate agents ;
b) assessing said biological sample ;
   comparing the sample in step b) to one in the absence and/or presence of fluoxetine or a derivative thereof as defined above.

The above methods may optionally further comprise the step d) of determining whether the candidate agent or combination of agents is treating myocardial infarction, based upon the comparison in step c).

The biological sample comprising a myocardial infarct can be assessed for example by histological analysis, so as to measure the size of ischemic and necrotic muscular heart tissue, or by any other suitable method as described above. Said biological sample is preferably a cardiac biopsy.

The present invention will be better understood in the light of the following detailed description of experiments, including examples. Nevertheless, the skilled artisan will appreciate that this detailed description is not limitative and that various modifications, substitutions, omissions, and changes may be made without departing from the scope of the invention.

### BRIEF DESCRIPTION OF THE DRAWINGS

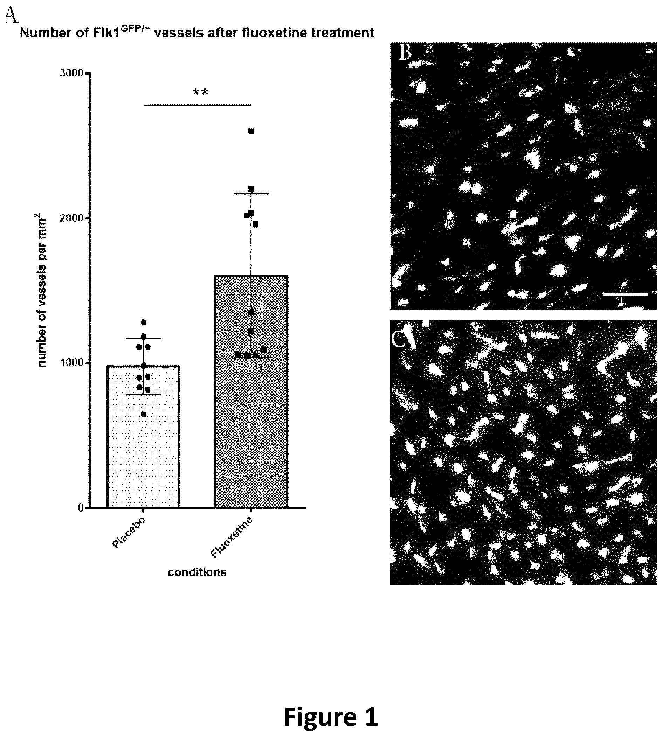

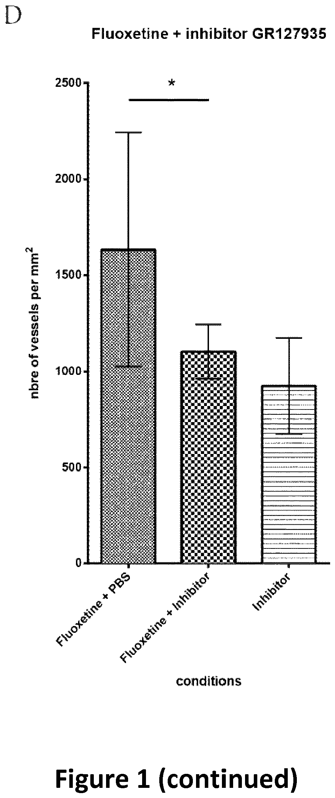

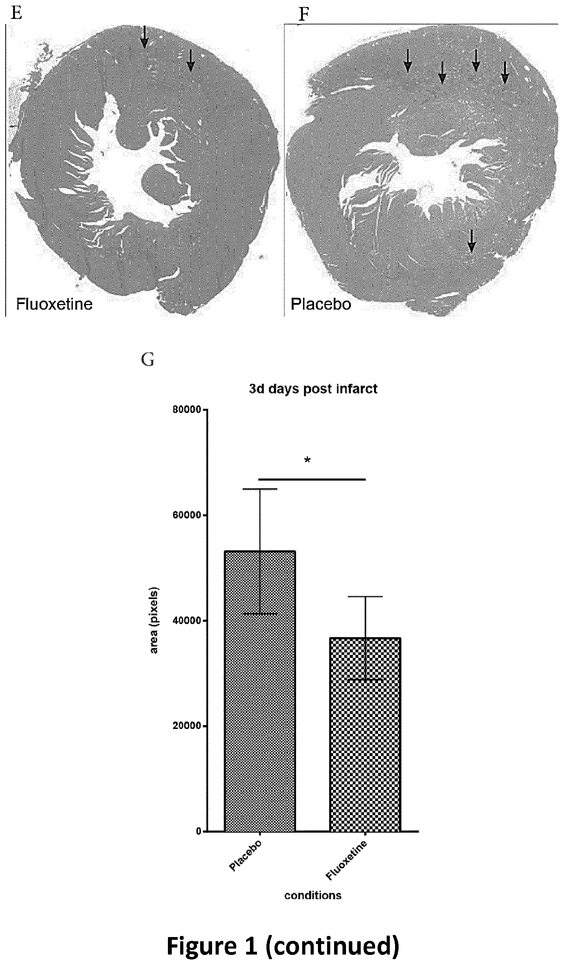

### Figure 1. Fluoxetine improves the phenotype of ischemic heart diseased mice

**(a)** Number of vessels counted per mm² in cryo-sectioned heart from *Flk1*^{*GFP*/+} mice treated with fluoxetine 6 weeks PO and compared with placebo. Counting was done based on the endogenous fluorescence. **(b-c)** Cryo-sections of the heart of *Flk1*^{*GFP*/+} mice treated with placebo **(b)** or fluoxetine **(c).** Endogenous GFP levels are shown. **(d)** Number of vessels (counted with endogenous GFP from *Flk1*^{*GFP*/+} mouse) in fluoxetine, fluoxetine and 5HT1B inhibitor, inhibitor alone per mm² in the heart. **(e-f)** Haematoxylin and eosin staining of heart of the placebo and the fluoxetine treated mice. **(g)** Quantification of the infarcted area with or without fluoxetine treatment. The units are displayed in pixels.

### EXAMPLES

### Fluoxetine improves the heart after myocardial infarct

### 1. MATERIAL AND METHODS

### 1.1. Mice injection and injury

Animals were anesthetized with ketamine (Imalgene1000 100mg/Kg Merial) and Xylazine (Rompun2% 20mg/Kg Bayer) prior to injury. Animals were hydrated and treated with analgesic (Buprenorphin Axience 0.3mg/kg) twice a day for 4 days following injury. All protocols were reviewed by the Institut Pasteur, the competent authority, for compliance with the French and European regulations on Animal Welfare and with Public Health Service recommendations. This project has been reviewed and approved (# 2013-0044) by the Institut Pasteur ethic committee (C2EA 89 - CETEA).

Among the mice tested, *Flk1*^{*GFP*/+} mice, in which green fluorescent protein (GFP) is targeted in VEGF-receptor-2 gene locus, and which exhibits a bright GFP signal in all endothelial cells, were kindly provided by Alexander Medvinsky (Institute for Stem Cell Research, University of Edinburgh, Edinburgh, UK).

### 1.2. Histological analysis

Hearts were carefully dissected and snap frozen in liquid-nitrogen-cooled OCT for a few minutes and stored at -80°C prior to cryosectioning (10µm sections). Sections were kept at room temperature overnight before staining. Sections were then rehydrated in PBS for 10 minutes and fixed in 10% formalin for 3 minutes. The sections were then routinely stained with haematoxylin and eosin (HE) using an automated stain machine or manually with red sirius.

The slides were assessed by double blinding and automated when possible.

### 1.3. Immunostainings

Immunostaining was performed on cryosections fixed with 4% paraformaldehyde (PFA EMS#15710) in cold PBS, permeabilized with 0.5% Triton X-100 20 min at room temperature, washed, and blocked with 10% BSA for 30 min. Sections were incubated with primary antibodies overnight at 4°C and with Alexa-conjugated secondary antibodies 1/250 and Hoechst for 45 minutes. Sections were then analysed using an automated axioscan (Zeiss) or inverted Observer.Z1 Apotome (Zeiss). For apoptosis assessment, cells were collected in 2% serum, spun on polyD-lysine (Sigma-Aldrich#P6407), and immediately fixed with PFA 4%.

### 1.4. Image Analysis

For image analysis (fibrosis quantification) ImageJ 1.46r software was using between 10 different photos randomly taken per section and 3 sections minimum per experimental group. The pictures were converted in a binary image and the pixel values then collected. For fibre size, the sections were immunostained with rabbit anti Laminin (Sigma-Aldrich #L9393) diluted at 1/200, overnight at 4°C. Secondary Donkey ant Rabbit 488 (DL488 Jacksonlmmuno #711486152) were used at 1/200 45 minutes at room temperature. The fibre perimeter was done automatically by using Pixcavator® software.

### 1.5. Statistical analysis

Statistical analysis was performed using GraphPad Prism software using appropriate tests (non-parametric Mann-Whitney unless specified) and a minimum of 95% confidence interval for significance; p values indicated on figures are < 0.05 (*), < 0.01 (**), and < 0.001 (***). Figures display average values of all animals tested ± SD

### 2. RESULTS

### 2.1. Fluoxetine treatment reduces deleterious effects of ischemia in heart

Acute myocardial infarction is a major cause of morbidity and mortality worldwide (Murray and Lopez, 1997). Prompt restoration of coronary blood flow in the infarct-related artery is nowadays the most effective strategy for reducing myocardial infarct size and improving disease outcome (Hausenloy et al., 2012; Yellon and Hausenloy, 2007).

I.P (intraperitoneal) or P.O (per os) fluoxetine was delivered for 6 weeks to *Flk1*^{*GFP*/+} mice at 18mg/Kg and the vessel number in the heart was investigated. An increase in vessel number was observed when compared with placebo. 932.9±186 vessels were detected in the placebo, 1634±608 vessels (p=0.017) in the fluoxetine 6 weeks IP treated mice and 1474±375 vessels (p=0.03) in the 6 weeks PO treated mice **(****Figure 1a****-c).**

When inhibiting the 5-HT1BR with GR127935 hydrochloride inhibitor in osmotic pump together with fluoxetine treatment, no increase in the vessel number in the heart was observed (1697±702 vessels per mm² in fluoxetine and PBS treated mice *vs.* 1104±141 vessels per mm², p=0.06 in fluoxetine and inhibitor treated mice **Figure 1d**).

In order to investigate whether the increase in vessel number could protect the heart after a myocardial infarct, ischemia reperfusion was performed to C57BI/6 mice treated with fluoxetine for 6 weeks. First and as expected, a bigger infarcted area was observed in treated mice, as the number of vessels was higher (5% of total area in control vs. 25% in treated mice). However, 3 days after infarct at the histological level the infarcted area decreased by 31% (53126±11849 pixels in the placebo vs. 36690±7884 pixels, p=0.02 in the fluoxetine treated mice **Figure 1e****-g**). Therefore, fluoxetine protected heart from ischemia.

### 2. DISCUSSION

These results show that the administration of fluoxetine increased the number of vessels in cardiac muscles. These results were confirmed using different approaches (genetics and immunostaining), routes of administration (intraperitoneal and *per os*) and different concentrations.

The inventors further showed that fluoxetine was acting via the serotonin receptors 5-HT1BR and that pharmaceutical delivery of 5-HT1BR inhibitor cancelled the effects of fluoxetine (on the increase number of vessels).

Crucially, 3 days after ischemia, the heart of fluoxetine treated animals was less infarcted with fewer lesions, demonstrating a protecting effect of fluoxetine.

### REFERENCES

Celermajer DS, Chow CK, Marijon E, Anstey NM and Woo KS (2012). J. Am. Coll. Cardiol.; 60:1207-1216.
Farmaco, 1989, 44, from p.683.
Hausenloy DJ, Boston-Griffiths EA and Yellon DM (2012). Cyclosporin A and cardioprotection: From investigative tool to therapeutic agent. Br. J. Pharmacol.; 165:1235-1245.
Kuroda N, Ohyama Y, Nakashima K, Nakashima K, and Akiyama S (1996). Chemical and Pharmaceutical Bulletin; 44(8): 1525-1529.
Laflamme MA, Zbinden S, Epstein SE and Murry CE (2007). Annu. Rev. Pathol.; 2: 307-339.
Mokrosz JL, Pietrasiewicz M, Duszynska B, and Cegla M (1992). J. Med. Chem.; 35 (13): 2369-2374.
Murray CJL. and Lopez AD (1997). Lancet; 349:1498-1504.
Nakaya K, Tanaka T, Shirataki Y, Shiozaki H, Funabiki K, Shibata K, Matsui M (2001). Bulletin of the Chemical Society of Japan.; 74(1): 173-177.
Romanelli MN, Manetti D, Scapecchi S, Borea PA, Dei S, Bartolini A, Ghelardini C, Gualtieri F, Guandalini L, and Varani K (2001). J Med Chem.; 44(23):3946-55.
Yellon DM and Hausenloy DJ (2007). N. Engl. J. Med.; 357:1121-1135.

## Claims

1. Fluoxetine or a derivative thereof, for use for reducing the size of a myocardial infarct in a patient suffering from myocardial infarction, wherein said derivative is fluoxetine which is modified to comprise one or more charged chemical moieties.

2. Fluoxetine or a derivative thereof for use according to claim 1, wherein said charged chemical moiety is a positively charged moiety.

3. Fluoxetine or a derivative thereof for use according to claim 1 or 2, wherein said charged moiety is a quaternary ammonium group.

4. Fluoxetine or a derivative thereof for use according to claim 3, wherein said quaternary ammonium group has the formula (I)
-(NR₁R₂R₃)⁺Z⁻ (I)
wherein
Z is an organic or inorganic anion; and
R₁, R₂ and R₃ are each independently selected from the group consisting of alkyl, aryl and cycloalkyl.

5. A pharmaceutical composition for use for reducing the size of a myocardial infarct in a patient suffering from myocardial infarction, wherein said composition comprises fluoxetine or a derivative thereof as defined in any one of claims 1 to 4 and at least one pharmaceutically acceptable excipient.

6. The composition for use according to claim 5, further comprising at least one active agent.

7. The composition for use according to claim 6, wherein said active agent is selected from the group consisting of beta-blockers, antithrombotics, trinitrine, vasodilatators, angiotensin-converting-enzyme inhibitors, angiotensin-receptor blockers, L-carnitine, lidocaine, calcium channel inhibitors, non-steroidal anti-inflammatory drugs (NSAIDs), therapeutic cells, and growth factors.

8. Fluoxetine or a derivative thereof for use for reducing the size of a myocardial infarct as defined in any one of claims 1 to 4 and an active agent as defined in claim 6 or 7, as a combined preparation for simultaneous, separate or sequential administration for reducing the size of a myocardial infarct in a subject suffering from myocardial infarction.

9. An *in vitro* screening method for identifying an agent or combination of agents treating myocardial infarction, comprising the steps of:
a) contacting a biological sample comprising a myocardial infarct with a candidate agent or combination of candidate agents;
b) assessing said biological sample ;
c) comparing the sample in step b) to one in the absence and/or presence of fluoxetine or a derivative thereof as defined in any one of claims 1 to 4.

## Patentansprüche

1. Fluoxetin oder ein Derivat davon zur Verwendung bei der Verminderung der Größe eines Myokardinfarkts bei einem Patienten, der an einem Myokardinfarkt leidet, wobei das Derivat Fluoxetin ist, das modifiziert ist, um eine oder mehrere geladene chemische Einheiten zu umfassen.

2. Fluoxetin oder ein Derivat davon zur Verwendung nach Anspruch 1, wobei die geladene chemische Einheit eine positiv geladene Einheit ist.

3. Fluoxetin oder ein Derivat davon zur Verwendung nach Anspruch 1 oder 2, wobei die geladene Einheit eine quaternäre Ammoniumgruppe ist.

4. Fluoxetin oder ein Derivat davon zur Verwendung nach Anspruch 3, wobei die quaternäre Ammoniumgruppe die Formel (I) aufweist
-(NR₁R₂R₃)⁺Z⁻ (I)
wobei
Z ein organisches oder anorganisches Anion ist; und
R₁, R₂ und R₃ jeweils unabhängig aus der Gruppe ausgewählt sind, die aus Alkyl, Aryl und Cycloalkyl besteht.

5. Pharmazeutische Zusammensetzung zur Verwendung bei der Verminderung der Größe eines Myokardinfarkts bei einem Patienten, der an einem Myokardinfarkt leidet, wobei die Zusammensetzung Fluoxetin oder ein Derivat davon nach einem der Ansprüche 1 bis 4 und mindestens einen pharmazeutisch annehmbaren Hilfsstoff umfasst.

6. Zusammensetzung zur Verwendung nach Anspruch 5, die ferner mindestens einen Wirkstoff umfasst.

7. Zusammensetzung zur Verwendung nach Anspruch 6, wobei der Wirkstoff aus der Gruppe ausgewählt ist, die aus Betablockern, Antithrombotika, Trinitrin, Vasodilatatoren, Angiotensin-umwandelnden Enzyminhibitoren, Angiotensinrezeptorblockern, L-Carnitin, Lidocain, Calciumkanalinhibitoren, nichtsteroidalen Antirheumatika (NSAR), therapeutischen Zellen und Wachstumsfaktoren besteht.

8. Fluoxetin oder ein Derivat davon zur Verwendung bei der Verminderung der Größe eines Myokardinfarkts nach einem der Ansprüche 1 bis 4 und Wirkstoff nach Anspruch 6 oder 7 als ein Kombinationspräparat zur gleichzeitigen, getrennten oder aufeinanderfolgenden Verabreichung zur Verminderung der Größe eines Myokardinfarkts bei einem Subjekt, das an einem Myokardinfarkt leidet.

9. In-vitro-Screening-Verfahren zum Identifizieren eines Mittels oder einer Kombination von Mitteln, die Myokardinfarkte behandeln, das die folgenden Schritte umfasst:
a) In-Kontakt-Bringen einer biologischen Probe, die einen Myokardinfarkt umfasst, mit einem Kandidatenmittel oder einer Kombination von Kandidatenmitteln;
b) Bewerten der biologischen Probe;
c) Vergleichen der Probe in Schritt b) mit einer bei Nichtvorhandensein und/oder Vorhandensein von Fluoxetin oder eines Derivats davon nach einem der Ansprüche 1 bis 4.

## Revendications

1. Fluoxétine ou un dérivé de celle-ci, pour une utilisation afin de réduire la taille d'un infarctus du myocarde chez un patient souffrant d'un infarctus du myocarde, dans lequel ledit dérivé est la fluoxétine qui est modifiée de manière à comprendre un ou plusieurs groupes chimiques chargés.

2. Fluoxétine ou un dérivé de celle-ci pour une utilisation selon la revendication 1, dans lequel ledit groupe chimique chargé est un groupe chargé positivement.

3. Fluoxétine ou un dérivé de celle-ci pour une utilisation selon la revendication 1 ou 2, dans lequel ledit groupe chargé est un groupe ammonium quaternaire.

4. Fluoxétine ou un dérivé de celle-ci pour une utilisation selon la revendication 3, dans lequel ledit groupe ammonium quaternaire répond à la formule (I)
-(NR₁R₂R₃)⁺Z⁻ (I)
dans laquelle
Z est un anion organique ou inorganique ; et
chacun de R₁, R₂ et R₃ est indépendamment choisi dans l'ensemble constitué par alkyle, aryle et cycloalkyle.

5. Composition pharmaceutique pour une utilisation afin de réduire la taille d'un infarctus du myocarde chez un patient souffrant d'un infarctus du myocarde, laquelle composition comprend de la fluoxétine ou un dérivé de celle-ci tel que défini dans l'une quelconque des revendications 1 à 4 et au moins un excipient pharmaceutiquement acceptable.

6. Composition pour une utilisation selon la revendication 5, comprenant en outre au moins un principe actif.

7. Composition pour une utilisation selon la revendication 6, dans laquelle ledit principe actif est choisi dans l'ensemble constitué par les bêtabloquants, les antithrombotiques, la trinitrine, les vasodilatateurs, les inhibiteurs de l'enzyme de conversion de l'angiotensine, les bloqueurs des récepteurs de l'angiotensine, la L-carnitine, la lidocaïne, les inhibiteurs calciques, les anti-inflammatoires non stéroïdiens (AINS), les cellules thérapeutiques, et les facteurs de croissance.

8. Fluoxétine ou un dérivé de celle-ci, pour une utilisation afin de réduire la taille d'un infarctus du myocarde, telle que définie dans l'une quelconque des revendications 1 à 4, et un principe actif tel que défini dans la revendication 6 ou 7, sous la forme d'une préparation combinée pour administration simultanée, séparée ou séquentielle afin de réduire la taille d'un infarctus du myocarde chez un patient souffrant d'un infarctus du myocarde.

9. Procédé de criblage *in vitro* pour identifier un agent ou une combinaison d'agents traitant un infarctus du myocarde, comprenant les étapes de :
a) mise en contact d'un échantillon biologique comprenant un infarctus du myocarde avec un agent candidat ou une combinaison d'agents candidats ;
b) évaluation dudit échantillon biologique ;
c) comparaison de l'échantillon dans l'étape b) à un autre en l'absence et/ou en présence de fluoxétine ou d'un dérivé de celui-ci tel que défini dans l'une quelconque des revendications 1 à 4.
